(19) Europäisches Patentamt
European Patent Office
Office européen des brevets

(11) Veröffentlichungsnummer: **0 544 169 B1**

# (12) EUROPÄISCHE PATENTSCHRIFT

(45) Veröffentlichungstag der Patentschrift: **17.11.94**

(51) Int. Cl.5: **C07D 223/20**, C07D 405/12, A61K 31/55

(21) Anmeldenummer: **92119531.9**

(22) Anmeldetag: **16.11.92**

## (54) 6-Thiono-5,6-dihydro-dibenz[b,e]azepin-11-on-11-oxime und ihre Verwendung als retrovirale Mittel.

(30) Priorität: **27.11.91 DE 4138908**

(43) Veröffentlichungstag der Anmeldung:
**02.06.93 Patentblatt 93/22**

(45) Bekanntmachung des Hinweises auf die Patenterteilung:
**17.11.94 Patentblatt 94/46**

(84) Benannte Vertragsstaaten:
**AT BE CH DE DK ES FR GB GR IE IT LI LU MC NL PT SE**

(56) Entgegenhaltungen:
**EP-A- 0 419 861**
**EP-A- 0 429 987**
**US-A- 3 431 257**

(73) Patentinhaber: **BAYER AG**
**D-51368 Leverkusen (DE)**

(72) Erfinder: **Wild, Hanno, Dr.**
**Ausblick 128**
**W-5600 Wuppertal 1 (DE)**
Erfinder: **Roeben, Wolfgang, Dr.**
**Strässchen Siefen 30**
**W-5060 Bergisch Gladbach (DE)**
Erfinder: **Paessens, Arnold, Dr.**
**Stresemannstrasse 51**
**W-5657 Haan (DE)**
Erfinder: **Petersen-von Gehr, Jörg, Dr.**
**Bärendorferstrasse 74**
**W-4630 Bochum 1 (DE)**

EP 0 544 169 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft 6-Thiono-dibenz[b,e]azepine, ein Verfahren zu ihrer Herstellung und ihre Verwendung als retrovirale Mittel.

Es ist bereits bekannt, daß substituierte 5,6-Dihydro-dibenz[b,e]azepin-6,11-dion-11-oxime eine psychotropische Wirkung besitzen.

Die vorliegende Erfindung betrifft jetzt 6-Thiono-dibenz[b,e]azepine der allgemeinen Formel (I),

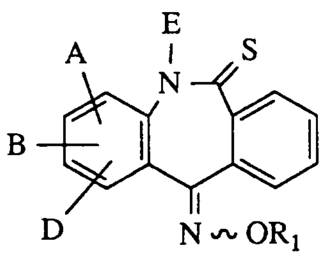

(I)

in welcher

| | |
|---|---|
| A, B und D | gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen, |
| E | für Wasserstoff oder für geradkettiges oder verzweigtes Alkyl mit bis zu 6 Kohlenstoffatomen steht, |
| $R^1$ | für Wasserstoff oder<br>für Cycloalkyl mit 3 bis 6 Kohlenstoffatomen oder für 2-Tetrahydropyranyl steht,<br>für geradkettiges oder verzweigtes Acyl mit bis zu 8 Kohlenstoffatomen steht, oder<br>für geradkettiges oder verzweigtes Alkyl oder Alkenyl mit jeweils bis zu 10 Kohlenstoffatomen steht, die gegebenenfalls durch Halogen, Hydroxy, Carboxy, geradkettiges oder verzweigtes Alkoxycarbonyl mit bis zu 6 Kohlenstoffatomen oder durch eine Gruppe der Formel $-NR^2R^3$ substituiert sind,<br>worin |
| $R^2$ und $R^3$ | gleich oder verschieden sind und Wasserstoff, geradkettiges oder verzweigtes Alkyl mit bis zu 8 Kohlenstoffatomen oder Phenyl bedeuten, oder |
| $R^2$ und $R^3$ | gemeinsam mit dem Stickstoffatom einen 5- bis 7-gliedrigen, gesättigten oder ungesättigten, heterocyclischen Ring mit bis zu 2 weiteren Heteroatomen aus der Reihe S, N oder O bilden,<br>oder dieses für $R^1$ stehende Alkyl oder Alkenyl durch Phenyl substituiert ist, das seinerseits bis zu 5-fach gleich oder verschieden durch Halogen substituiert sein kann |

und deren physiologisch unbedenklichen Salze.

Physiologisch unbedenkliche Salze der 6-Thiono-dibenz[b,e]azepine können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Heterocyclus steht im allgemeinen für eine 5- bis 7-gliedrigen, bevorzugt 5- bis 6-gliedrigen, gesättigten oder ungesättigten Ring, der als Heteroatome bis zu 2 Sauerstoff-, Schwefel- und/oder Stickstoffatome enthalten kann. Bevorzugt sind 5- und 6-gliedrige Ringe mit einem Sauerstoff-, Schwefel- und/oder bis zu 2 Stickstoffatomen. Besonders bevorzugt werden genannt: Pyrrolyl, Pyrazolyl, Pyridyl, Pyrimidyl, Pyrazinyl, Pyridazinyl, Thiazolyl, Oxazolyl, Imidazolyl, Isoxazolyl, Pyrrolidinyl, Piperidinyl, Piperazinyl, Tetrazolyl oder Morpholinyl.

Die erfindungsgemäßen Verbindungen können in stereoisomeren Formen existieren, die sich entweder wie Bild und Spiegelbild (Enantiomere), oder die sich nicht wie Bild und Spiegelbild (Diastereomere) verhalten. Die Erfindung betrifft sowohl die Antipoden als auch die Racemformen sowie die Diastereomerengemische. Die Racemformen lassen sich ebenso wie die Diastereomeren in bekannter Weise in die stereoisomer einheitlichen Bestandteile trennen [vgl. E.L. Eliel, Stereochemistry of Carbon Compounds, McGraw Hill, 1962].

Bei dem Rest der allgemeinen Formel (II)

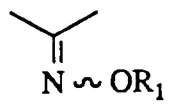

(II)

kann die C = N-Doppelbindung sowohl die E- als auch die Z-Konfiguration besitzen, bzw. es können E/Z-Gemische vorliegen.

Bevorzugt sind Verbindungen der allgemeinen Formel (I),
in welcher

A, B und D gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen,

E für Wasserstoff oder für geradkettiges oder verzweigtes Alkyl mit bis zu 4 Kohlenstoffatomen steht,

$R^1$ für Wasserstoff oder
für Cyclopropyl, Cyclopentyl, Cyclohexyl oder 2-Tetrahydropyranyl steht, oder
für geradkettiges oder verzweigtes Acyl mit bis zu 8 Kohlenstoffatomen steht, oder
für geradkettiges oder verzweigtes Alkyl oder Alkenyl mit jeweils bis zu 6 Kohlenstoffatomen steht, die gegebenenfalls durch Fluor, Hydroxy, Carboxy, geradkettiges oder verzweigtes Alkoxycarbonyl mit bis zu 4 Kohlenstoffatomen oder durch eine Gruppe der Formel $-NR^2R^3$ substituiert sind,
worin

$R^2$ und $R^3$ gleich oder verschieden sind und Wasserstoff oder geradkettiges oder verzweigtes Alkyl mit bis zu 6 Kohlenstoffatomen bedeuten,
oder

$R^2$ und $R^3$ gemeinsam mit dem Stickstoffatom einen Morpholin- oder Piperazinring bilden,
oder dieses für $R^1$ stehende Alkyl durch Phenyl substituiert ist, das seinerseits bis zu 5-fach gleich oder verschieden durch Fluor, Chlor oder Brom substituiert sein kann

und deren physiologisch unbedenklichen Salze.

Besonders bevorzugt sind Verbindungen der allgemeinen Formel (I),
in welcher

A, B und D gleich oder verschieden sind und für Wasserstoff, Fluor, Chlor oder für geradkettiges oder verzweigtes Alkyl oder Alkoxy mit jeweils bis zu 4 Kohlenstoffatomen stehen,

E für Wasserstoff, Methyl oder Ethyl steht,

$R^1$ für Wasserstoff oder
für Cyclopropyl oder 2-Tetrahydropyranyl steht, oder
für geradkettiges oder verzweigtes Acyl mit bis zu 4 Kohlenstoffatomen steht, oder
für geradkettiges oder verzweigtes Alkyl oder Alkenyl mit jeweils bis zu 6 Kohlenstoffatomen steht, die gegebenenfalls durch Hydroxy, Carboxy, Fluor, Methoxycarbonyl, Ethoxycarbonyl, Propoxycarbonyl oder durch eine Gruppe der Formel $-NR^2R^3$ substituiert sind,
worin

$R^2$ und $R^3$ gleich oder verschieden sind und Wasserstoff oder geradkettiges oder verzweigtes Alkyl mit bis zu 4 Kohlenstoffatomen bedeuten,
oder

$R^2$ und $R^3$ gemeinsam mit dem Stickstoffatom einen Morpholinring bilden,
oder dieses für $R^1$ stehende Alkyl durch Phenyl substituiert ist, das seinerseits bis zu 5-fach gleich oder verschieden durch Fluor oder Chlor substituiert sein kann

und deren physiologisch unbedenklichen Salze.

Außerdem wurde ein Verfahren zur Herstellung der erfindungsgemäßen Verbindungen der allgemeinen Formel (I) gefunden, dadurch gekennzeichnet, daß man
Verbindungen der allgemeinen Formel (III)

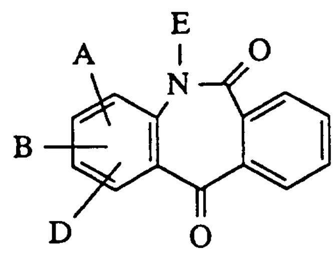

(III)

in welcher

A, B, D und E die oben angegebene Bedeutung haben,

zunächst mit Hydroxylaminen der allgemeinen Formel (IV)

$H_2N\text{-}O\text{-}R^1$ (IV)

in welcher

$R^1$ die oben angegebene Bedeutung hat,

in inerten Lösemitteln, gegebenenfalls in Anwesenheit einer Base umsetzt und anschließend mit Lawesson's Reagenz die der Gruppe -N-E benachbarte Carbonylin eine Thiocarbonylfunktion überführt, und gegebenenfalls die Substituenten A, B, D und $R^1$ nach üblichen chemischen Methoden variiert,

und im Fall, daß E nicht Wasserstoff bedeutet, ebenfalls nach bekannten Methoden eine Alkylierung durchführt.

Das erfindungsgemäße Verfahren kann durch folgendes Formelschema bespielhaft erläuert werden:

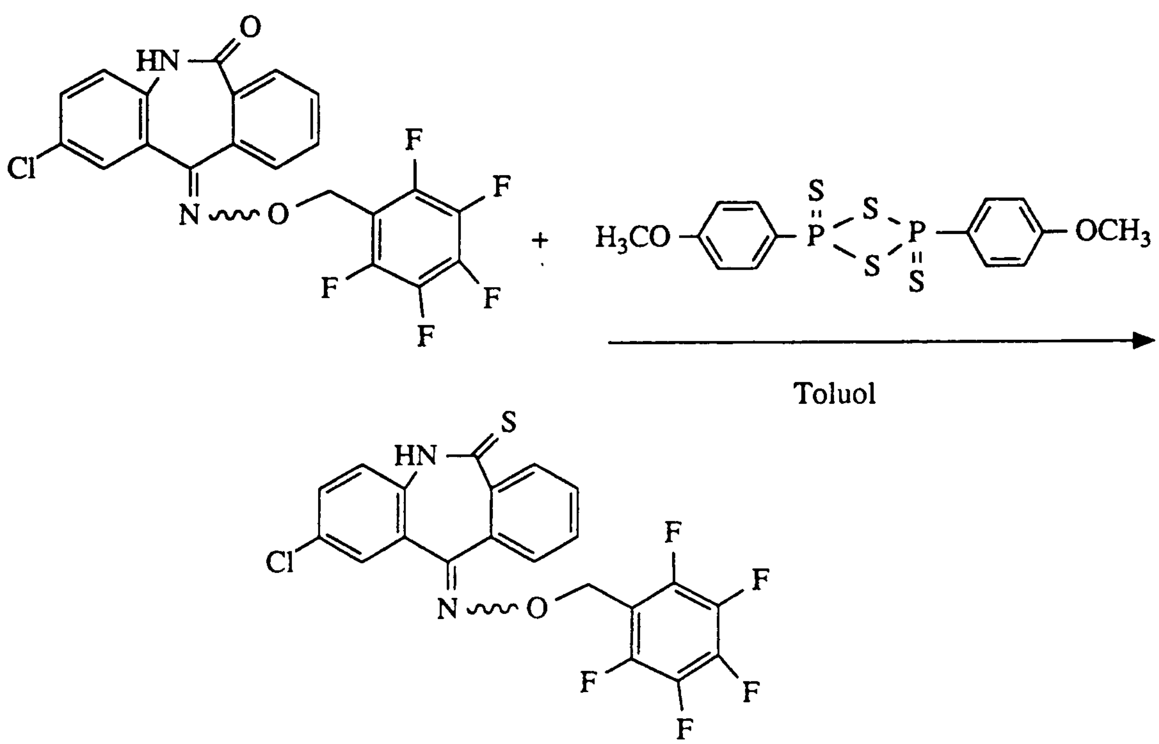


Als Lösemittel für die beiden Verfahrensschritte eignen sich die üblichen organischen Lösemittel, die sich unter den Reaktionsbedingungen nicht verändern. Hierzu gehören bevorzugt Ether wie Diethylether, Dioxan, Tetrahydrofuran, Glykoldimethylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt sind für den 1.

Reaktionsschritt Pyridin, für den zweiten Toluol.

Als Basen eignen sich die üblichen basischen Verbindungen. Hierzu gehören vorzugsweise Alkali- oder Erdalkalicarbonate wie Natriumcarbonat, Kaliumcarbonat, oder Alkalialkoholate wie beispielsweise Natriummethanolat oder -ethanolat, Kaliummethanolat oder -ethanolat oder Kalium-tert.butylat, oder organische Amine wie Benzyltrimethylammoniumhydroxid, Tetrabutylammoniumhydroxid, Pyridin, Triethylamin oder N-Methylpiperidin.

Die Verfahrensschritte werden im allgemeinen in einem Temperaturbereich von +30°C bis +150°C, bevorzugt von +50°C bis +120°C durchgeführt.

Im allgemeinen wird bei Normaldruck gearbeitet. Es ist aber auch möglich, das Verfahren bei Unterdruck oder bei Überdruck durchzuführen (z.B. in einem Bereich von 0,5 bis 5 bar).

Als Lösemittel für die Alkylierung ($E \neq H$) eignen sich ebenfalls übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Hexamethylphosphorsäuretriamid, Acetonitril, Aceton oder Nitromethan. Ebenso ist es möglich, Gemische der genannten Lösemittel zu verwenden. Bevorzugt ist Aceton.

Die Alkylierung wird in den oben aufgeführten Lösemitteln bei Temperaturen von 0°C bis +150°C, vorzugsweise bei Raumtemperaturen bis +100°C, bei Normaldruck durchgeführt.

Die Verbindungen der allgemeinen Formel (III) sind an sich bekannt oder können nach üblichen Methoden hergestellt werden [vgl.z.B. US 34 31 257].

Auch die Hydroxylamine der allgemeinen Formel (IV) sind bekannt oder können nach literaturbekannten Verfahren hergestellt werden.

Die hier beschriebenen Inhibitoren sind Inhibitoren der Reversen Transkriptase und sind als solche für alle Zwecke einsetzbar, für die Enzyminhibitoren geeignet sind. Dies ist zum Beispiel der Einsatz in der Diagnostik um die Präzision und Selektivität von Enzymaktivitätsmessungen zu verbessern. Bei der Affinitätschromätographie können sie als Afinitätslabel dienen und in der Forschung kann man sie zur Aufklärung von Reaktionsmechanismen enzymatischer Reaktionen verwenden.

Darüber hinaus wurde überraschend gefunden, daß die erfindungsgemäßen Verbindungen der allgemeinen Formel (I) eine außerordentlich starke Wirkung gegen Retroviren besitzen. Sie zeigen Wirkung in Lentivirus-infizierten Zellkulturen. Dies konnte am Beispiel des HIV-Virus gezeigt werden.

HIV-Infektion in Zellkultur

Der HIV-Test wurde mit geringen Modifikationen nach der Methode von Pauwels et al. [vgl. Journal of Virological Methods 20, (1988), 309-321] durchgeführt.

Normale menschliche Blutlymphozyten (PBL's) wurden über Ficoll-Hypaque angereichert und im RPMI 1640, 20% fötales Kälberserum mit Phythaemagglutinin (90 µg/ml) und Interleukin-2 (40 U/ml) stimutiert. Zur Infektion mit dem infektiösen HIV wurden PBL's pelletiert und das Zellpellet wurde anschließend in 1 mi HIV-Virusadsorptionslösung suspendiert und 1 Stunde bei 37°C inkubiert.

Alternativ wurden HIV-suszeptible H9-Zellen anstelle von normalen menschlichen Blutlymphozyten zur Testung der antiviralen Effekte der erfindungsgemäßen Verbindungen eingesetzt.

Die Virusadsorptionslösung wurde zentrifugiert und das infizierte Zellpellet in Wachstumsmedium aufgenommen, so daß $1 \times 10^5$ Zellen pro ml eingestellt waren. Die derart infizierten Zellen wurden zu $1 \times 10^4$ Zelle/Napf in die Näpfe von 96er Mikrotiterplatten pipettiert.

Die erste vertikale Reihe der Mikrotiterplatte enthielt nur Wachstumsmedium und Zellen, die nicht infiziert, aber ansonsten genauso wie oben beschrieben, behandelt worden waren (Zellkontrolle). Die zweite vertikale Reihe der Mikrotiterplatte erhielt nur HIV-infizierte Zellen (Viruskontrolle) in Wachstumsmedium. Die übrigen Näpfe enthielten die erfindungsgemäßen Verbindungen in unterschiedlichen Konzentrationen, ausgehend von den Näpfen der 3. vertikalen Reihe der Mikrotiterplatte, von der die Prüfsubstanzen in 2er Schritten $2^{10}$fach verdünnt wurden.

Die Testansätze wurden so lange bei 37°C inkubiert, bis in der unbehandelten Viruskontrolle die für das HIV typische Syncytienbildung auftrat (zwischen Tag 3 und 6 nach Infektion), die dann mikroskopisch ausgewertet wurde. In der unbehandelten Viruskontrolle resultierten unter diesen Testbedingungen etwa 20 - 50 Syncytien, währen die unbehandelte Zellkontrolle keine Syncytien aufwies.

Die $IC_{50}$-Werte wurden als die Konzentration der behandelten und infizierten Zellen ermittelt, bei der 50% (ca. 10 - 20 Syncytien) der virusinduzierten Syncytien durch die Behandlung mit der erfindungsgemäßen Verbindung unterdrückt waren.

Es wurde nun gefunden, daß die erfindungsgemäßen Verbindungen HIV infizierte Zellen vor der virusinduzierten Zellzerstörung schützen.

Tabelle I

| Bsp.-Nr. | $IC_{50}$ (µM) |
|---|---|
| 2 | 1,5 |
| 3 | 0,38 |
| 4 (Vergleich) | 0,38 |
| BIRG 587 [J. Med. Chem.34 2231,(1991)] | 0,09 |

Die erfindungsgemäßen Verbindungen stellen wertvolle Wirkstoffe zur Behandlung und Prophylaxe von Erkrankungen, hervorgerufen durch Retroviren, in der Human- und Tiermedizin dar.

Als Indikationsgebiete in der Humanmedizin können beispielsweise genannt werden:

1.) Die Behandlung und Prophylaxe von menschlichen Retrovirusinfektionen.

2.) Für die Behandlung oder Prophylaxe von HIV I (Virus der humanen Immundefizienz; früher HTLV III/LAV genannt) und HIV II verursachten Erkrankungen (AIDS) und den damit assoziierten Stadien wie ARC (AIDS related complex) und LAS (Lymphadenopathie-Syndrom) sowie der durch dieses Virus verursachten Immunschwäche und Encephalopathie.

3.) Für die Behandlung oder die Prophylaxe einer HTLV-I oder HTLV-II Infektion.

4.) Für die Behandlung oder die Prophylaxe des AIDS-carrier Zustandes (AIDS-Überträger-Zustand).

Als Indikationen in der Tiermedizin können beispielsweise angeführt werden:

Infektionen mit

a) Maedivisna (bei Schafen und Ziegen)
b) progressivem Pneumonievirus (PPV) (bei Schafen und Ziegen)
c) caprine arthritis encephalitis Virus (bei Schafen und Ziegen)
d) Zwoegerziekte Virus (bei Schafen)
e) infektiösem Virus der Anämie (des Pferdes)
f) Infektionen verursacht durch das Katzenleukämievirus
g) Infektionen verursacht durch das Virus der Katzen-Immundefizienz (FIV)
h) Infektionen verursacht durch das Virus der Affen-Immundefizienz (SIV)

Bevorzugt werden aus dem Indikationsgebiet in der Humanmedizin die oben aufgeführten Punkte 2,3 und 4.

Zur vorliegenden Erfindung gehören pharmazeutische Zubereitungen, die neben nicht-toxischen, inerten pharmazeutisch geeigneten Trägerstoffen eine oder mehrere Verbindungen der Formel (I) enthalten oder die aus einem oder mehreren Wirkstoffen der Formel (I) bestehen, sowie Verfahren zur Herstellung dieser Zubereitungen.

Die Wirkstoffe der Formel (I) sollen in den oben aufgeführten pharmazeutischen Zubereitungen, vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den Verbindungen der Formel (I) auch weitere pharmazeutische Wirkstoffe enthalten.

Die Herstellung der oben aufgeführten pharmazeutischen Zubereitungen erfolgt in üblicher Weise nach bekannten Methoden, z.B. durch Mischen des oder der Wirkstoffe mit dem oder den Trägerstoffen.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,1 bis etwa 200, vorzugsweise 1 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die Wirkstoffe vorzugsweise in Mengen von etwa 1 bis etwa 80, insbesondere 1 bis 30 mg/kg Körpergewicht. Es kann jedoch erforderlich sein, von den genannten Dosierungen abzuweichen, und zwar in Abhängigkeit von der Art und dem Körpergewicht des zu behandelnden Objekts, der Art und der Schwere der Erkrankung, der Art der Zubereitung und der Applikation des Arzneimittels sowie dem Zeitraum bzw. Intervall, innerhalb welchem die Verabreichung erfolgt.

Herstellungsbeispiele

Beispiel 1

(E/Z)-2-Chlor-11-(pentafluorphenyl)methyloxyimino-6-thiono-5,6-dihydro-11H-dibenz[b,e]azepin

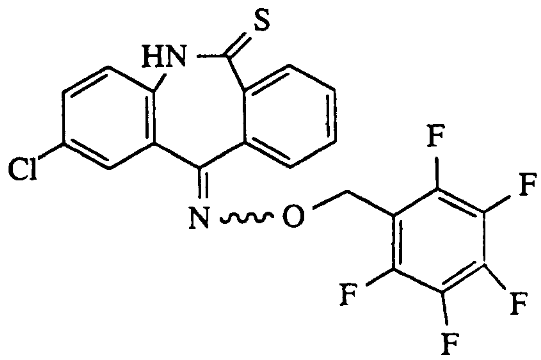

200 mg (0,44 mmol) (E/Z)-2-Chlor-11-(pentafluorphenyl)methyl-oxyimino-6-oxo-5,6-dihydro-11H-dibenz[b,e]-azepin in 3,5 ml Toluol werden mit 89 mg (0,22mmol) Lawesson's Reagenz versetzt und 3 h unter Rückfluß erhitzt. Anschließend wird eingeengt und der Rückstand an Kieselgel mit Toluol/Petrolether 20:1 gereinigt.
Ausbeute: 163 mg
$^{1}$H-NMR ($CDCl_3$) + DMSO): $\delta$ = 5,30 (s, 2H); 7,20 - 7,50 (m, 6H); 8,27 (d, J = 8 Hz, 1H); 12,47 (s, NH).

In Analogie zur Vorschrift des Beispiels 1 werden die in Tabelle 1 aufgeführten Beispiele hergestellt:

**Tabelle 1:**

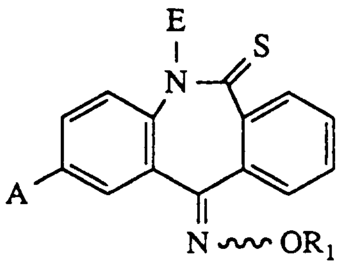

| Bsp.-Nr. | A | E | $R^1$ | E/Z |
|---|---|---|---|---|
| 2 | Cl | H | $-(CH_2)_2N(CH_3)_2$ | 1:1 |
| 3 | Cl | H | 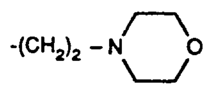 | 1:1 |
| 4 | Cl | H | $-CH_3$ | 1:1 |
| 5 | Cl | $-CH_3$ | $-CH_3$ | 1:1 |
| 6 | Cl | H | $-C_2H_5$ | 1:1 |
| 7 | Cl | H | $-C(CH_3)_3$ | 1:1 |
| 8 | Cl | H | $-CH_2-CH=CH_2$ | 1:1 |

Fortsetzung Tabelle 1:

| Bsp.-Nr. | A | E | $R^1$ | E/Z |
|---|---|---|---|---|
| 9 | Cl | H | 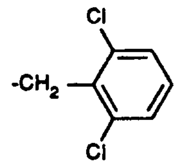 | 1:1 |
| 10 | Cl | H | $-CH_2-CO_2CH_3$ | 1:1 |
| 11 | Cl | $-CH_3$ | $-CH_2-CO_2CH_3$ | 1:1 |
| 12 | Cl | H | $-CH_2-CO_2-C_2H_5$ | 1:1 |
| 13 | Cl | $-CH_3$ | $-CH_2CO_2C_2H_5$ | 1:1 |
| 14 | Cl | H | $-(CH_2)_4CO_2C_2H_5$ | 1:1 |
| 15 | Cl | H | $-CO-CH_3$ | 1:1 |

**Patentansprüche**

**1.** 6-Thiono-dibenz[b,e]azepine der allgemeinen Formel (I),

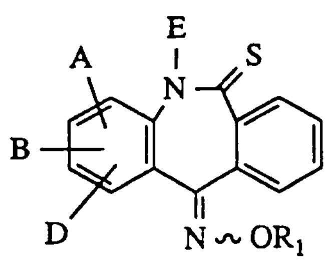

(I)

in welcher

A, B und D gleich oder verschieden sind und für Wasserstoff, Amino, Nitro, Halogen, Cyano, Hydroxy, Trifluormethyl, Trifluormethoxy oder für geradkettiges oder verzweigtes Alkyl oder Alkoxy mit jeweils bis zu 8 Kohlenstoffatomen stehen,

E für Wasserstoff oder für geradkettiges oder verzweigtes Alkyl mit bis zu 6 Kohlenstoffatomen steht,

| | |
|---|---|
| $R^1$ | für Wasserstoff oder<br>für Cycloalkyl mit 3 bis 6 Kohlenstoffatomen oder für 2-Tetrahydropyranyl steht,<br>für geradkettiges oder verzweigtes Acyl mit bis zu 8 Kohlenstoffatomen steht, oder<br>für geradkettiges oder verzweigtes Alkyl oder Alkenyl mit jeweils bis zu 10 Kohlenstoffatomen steht, die gegebenenfalls durch Halogen, Hydroxy, Carboxy, geradkettiges oder vertweigtes Alkoxycarbonyl mit bis zu 6 Kohlenstoffatomen oder durch eine Gruppe der Formel $-NR^2R^3$ substituiert sind,<br>worin |
| $R^2$ und $R^3$ | gleich oder verschieden sind und Wasserstoff, geradkettiges oder verzweigtes Alkyl mit bis zu 8 Kohlenstoffatomen oder Phenyl bedeuten, oder |
| $R^2$ und $R^3$ | gemeinsam mit dem Stickstoffatom einen 5- bis 7-gliedrigen, gesättigten oder ungesättigten, heterocyclischen Ring mit bis zu 2 weiteren Heteroatomen aus der Reihe S, N oder O bilden,<br>oder dieses für $R^1$ stehende Alkyl oder Alkenyl durch Phenyl substituiert ist, das seinerseits bis zu 5-fach gleich oder verschieden durch Halogen substituiert sein kann |

und deren physiologisch unbedenklichen Salze.

**2.** Verbindungen der allgemeinen Formel (I) gemäß Anspruch 1, in welcher

| | |
|---|---|
| A, B und D | gleich oder verschieden sind und für Wasserstoff, Fluor, Chlor, Hydroxy, Trifluormethyl, Trifluormethoxy oder für geradkettiges oder verzweigtes Alkyl oder Alkoxy mit jeweils bis zu 6 Kohlenstoffatomen stehen, |
| E | für Wasserstoff oder für geradkettiges oder verzweigtes Alkyl mit bis zu 4 Kohlenstoffatomen steht, |
| $R^1$ | für Wasserstoff oder<br>für Cyclopropyl, Cyclopentyl, Cyclohexyl oder 2-Tetrahydropyranyl steht, oder<br>für geradkettiges oder verzweigtes Acyl mit bis zu 6 Kohlenstoffatomen steht, oder<br>für geradkettiges oder verzweigtes Alkyl oder Alkenyl mit jeweils bis zu 8 Kohlenstoffatomen steht, die gegebenenfalls durch Fluor, Hydroxy, Carboxy, geradkettiges oder verzweigtes Alkoxycarbonyl mit bis zu 4 Kohlenstoffatomen oder durch eine Gruppe der Formel $-NR^2R^3$ substituiert sind,<br>worin |
| $R^2$ und $R^3$ | gleich oder verschieden sind und Wasserstoff oder geradkettiges oder verzweigtes Alkyl mit bis zu 6 Kohlenstoffatomen bedeuten,<br>oder |
| $R^2$ und $R^3$ | gemeinsam mit dem Stickstoffatom einen Morpholin- oder Piperazinring bilden,<br>oder dieses für $R^1$ stehende Alkyl durch Phenyl substituiert ist, das seinerseits bis zu 5-fach gleich oder verschieden durch Fluor, Chlor oder Brom substituiert sein kann |

und deren physiologisch unbedenklichen Salze.

**3.** Verbindungen der allgemeinen Formel (I) gemäß Anspruch 1, in welcher

| | |
|---|---|
| A, B und D | gleich oder verschieden sind und für Wasserstoff, Fluor, Chlor oder für geradkettiges oder verzweigtes Alkyl oder Alkoxy mit jeweils bis zu 4 Kohlenstoffatomen stehen, |
| E | für Wasserstoff, Methyl oder Ethyl steht, |
| $R^1$ | für Wasserstoff oder<br>für Cyclopropyl oder 2-Tetrahydropyranyl steht, oder<br>für geradkettiges oder verzweigtes Acyl mit bis zu 4 Kohlenstoffatomen steht, oder<br>für geradkettiges oder verzweigtes Alkyl oder Alkenyl mit jeweils bis zu 6 Kohlenstoffatomen steht, die gegebenenfalls durch Hydroxy, Carboxy, Fluor, Methoxycarbonyl, Ethoxycarbonyl, Propoxycarbonyl oder durch eine Gruppe der Formel $-NR^2R^3$ substituiert sind,<br>worin |
| $R^2$ und $R^3$ | gleich oder verschieden sind und Wasserstoff oder geradkettiges oder verzweigtes Alkyl mit bis zu 4 Kohlenstoffatomen bedeuten,<br>oder |
| $R^2$ und $R^3$ | gemeinsam mit dem Stickstoffatom einen Morpholinring bilden,<br>oder dieses für $R^1$ stehende Alkyl durch Phenyl substituiert ist, das seinerseits bis zu |

5-fach gleich oder verschieden durch Fluor oder Chlor substituiert sein kann

und deren physiologisch unbedenklichen Salze.

**4.** Verfahren zur Herstellung der erfindungsgemäßen Verbindungen der allgemeinen Formel (I)nach Anspruch 1, dadurch gekennzeichnet, daß man

Verbindungen der allgemeinen Formel (III)

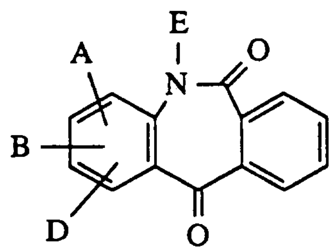

(III)

in welcher

A, B, D und E die oben angegebene Bedeutung haben,

zunächst mit Hydroxylaminen der allgemeinen Formel (IV)

$H_2N-O-R^1$ (IV)

in welcher

$R^1$ die oben angegebene Bedeutung hat,

in inerten Lösemitteln, gegebenenfalls in Anwesenheit einer Base umsetzt und anschließend mit Lawesson's Reagenz die der Gruppe -N-E benachbarte Carbonyl- in eine Thiocarbonylfunktion überführt, und gegebenenfalls die Substituenten A, B, D und $R^1$ nach üblichen chemischen Methoden variiert, und im Fall, daß E nicht Wasserstoff bedeutet, ebenfalls nach bekannten Methoden eine Alkylierung durchführt.

**5.** Arzneimittel enthaltend eine oder mehrere der Verbindungen aus den Ansprüchen 1 bis 4.

**6.** Verwendung der Verbindungen aus den Ansprüchen 1 bis 4 zur Herstellung von Arzneimitteln.

## Claims

**1.** 6-Thiono-dibenz[b,e]azepines of the general formula (I)

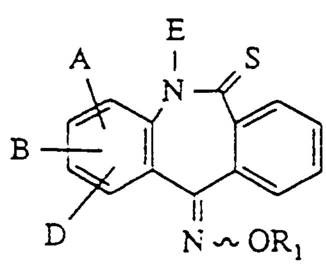

(I)

in which

A, B and D are identical or different and represent hydrogen, amino, nitro, halogen, cyano, hydroxyl, trifluoromethyl, trifluoromethoxy or straight-chain or branched alkyl or alkoxy each having up to 8 carbon atoms,

E represents hydrogen or straight-chain or branched alkyl having up to 6 carbon atoms,

$R^1$ represents hydrogen or

represents cycloalkyl having 3 to 6 carbon atoms or represents 2-tetrahydropyranyl,

represents straight-chain or branched acyl having up to 8 carbon atoms, or

represents straight-chain or branched alkyl or alkenyl each having up to 10 carbon

atoms, each of which is optionally substituted by halogen, hydroxyl or carboxyl, by straight-chain or branched alkoxycarbonyl having up to 6 carbon atoms or by a group of the formula $-NR^2R^3$,
in which
$R^2$ and $R^3$ are identical or different and denote hydrogen, straight-chain or branched alkyl having up to 8 carbon atoms or phenyl, or
$R^2$ and $R^3$ , together with the nitrogen atom, form a 5- to 7-membered, saturated or unsaturated heterocyclic ring having up to 2 further heteroatoms from the series comprising S, N and O,
or this alkyl or alkenyl representing $R^1$ is substituted by phenyl which in turn can be subtituted up to 5 times by identical or different halogen
and their physiologically acceptable salts.

**2.** Compounds of the general formula (I) according to Claim 1,
in which
A, B and D are identical or different and represent hydrogen, fluorine, chlorine, hydroxyl, trifluoromethyl, trifluoromethoxy or straight-chain or branched alkyl or alkoxy each having up to 6 carbon atoms,
E represents hydrogen or straight-chain or branched alkyl having up to 4 carbon atoms,
$R^1$ represents hydrogen or
represents cyclopropyl, cyclopentyl, cyclohexyl or 2-tetrahydropyranyl, or
represents straight-chain or branched acyl having up to 6 carbon atoms, or
represents straight-chain or branched alkyl or alkenyl each having up to 8 carbon atoms, each of which can optionally be substituted by fluorine, hydroxyl or carboxyl, by straight-chain or branched alkoxycarbonyl having up to 4 carbon atoms or by a group of the formula $-NR^2R^3$,
in which
$R^2$ and $R^3$ are identical or different and denote hydrogen or straight-chain or branched alkyl having up to 6 carbon atoms,
or
$R^2$ and $R^3$ , together with the nitrogen atom, form a morpholine or piperazine ring,
or this alkyl representing $R^1$ is substituted by phenyl which in turn can be substituted up to 5 times by identical or different fluorine, chlorine or bromine
and their physiologically acceptable salts.

**3.** Compounds of the general formula (I) according to Claim 1,
in which
A, B and D are identical or different and represent hydrogen, fluorine, chlorine or straight-chain or branched alkyl or alkoxy each having up to 4 carbon atoms,
E represents hydrogen, methyl or ethyl,
$R^1$ represents hydrogen or
represents cyclopropyl or 2-tetrahydropyranyl, or represents straight-chain or branched acyl having up to 4 carbon atoms, or
represents straight-chain or branched alkyl or alkenyl each having up to 6 carbon atoms, each of which is optionally substituted by hydroxyl, carboxyl, fluorine, methoxycarbonyl, ethoxycarbonyl or propoxycarbonyl or by a group of the formula $-NR^2R^3$,
in which
$R^2$ and $R^3$ are identical or different and denote hydrogen or straight-chain or branched alkyl having up to 4 carbon atoms,
or
$R^2$ and $R^3$ , together with the nitrogen atom, form a morpholine ring,
or this alkyl representing $R^1$ is substituted by phenyl which in turn can be substituted up to 5 times by identical or different fluorine or chlorine
and their physiologically acceptable salts.

**4.** Process for the preparation of the compounds according to the invention of the general formula (I) according to Claim 1, characterized in that
compounds of the general formula (III)

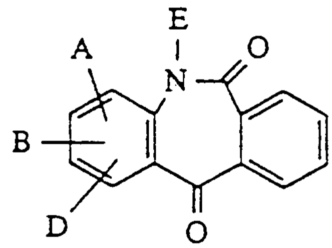

(III)

in which

A, B, D and E have the abovementioned meaning,

are first reacted with hydroxylamines of the general formula (IV)

$H_2N-O-R^1$ (IV)

in which

$R^1$ has the abovementioned meaning,

in inert solvents, if appropriate in the presence of a base and then the carbonyl function adjacent to the -N-E group is converted into a thiocarbonyl function using Lawesson's reagent, and, if appropriate, the substituents A, B, D and $R^1$ are varied according to customary chemical methods, and in the case in which E does not denote hydrogen, an alkylation is likewise carried out according to known methods.

**5.** Medicaments containing one or more of the compounds from Claims 1 to 3.

**6.** Use of the compounds, from Claims 1 to 3 for the production of medicaments.

**Revendications**

**1.** 6-Thionodibenz[b,e]azépines de formule générale (I)

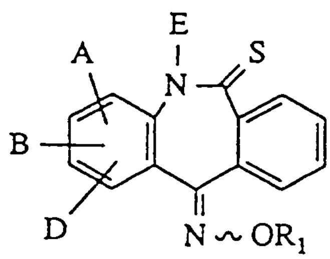

(I)

dans laquelle

A, B et D sont égaux ou différents et représentent de l'hydrogène, un groupe amino, nitro, un halogène, un groupe cyano, hydroxy, trifluorométhyle, trifluorométhoxy ou un groupe alkyle ou alkoxy linéaire ou ramifié ayant chacun jusqu'à 8 atomes de carbone,

E est de l'hydrogène ou un groupe alkyle linéaire ou ramifié ayant jusqu'à 6 atomes de carbone,

$R^1$ est de l'hydrogène ou représente un groupe cycloalkyle ayant 3 à 6 atomes de carbone ou 2-tétrahydropyrannyle, un groupe acyle linéaire ou ramifié ayant jusqu'à 8 atomes de carbone, ou bien un groupe alkyle ou alcényle linéaire ou ramifié ayant chacun jusqu'à 10 atomes de carbone, qui sont substitués le cas échéant par un halogène, un radical hydroxy, carboxy, alkoxycarbonyle linéaire ou ramifié ayant jusqu'à 6 atomes de carbone ou par un groupe de formule $-NR^2R^3$,

où

$R^2$ et $R^3$ sont égaux ou différents et représentent de l'hydrogène, un radical alkyle linéaire ou ramifié ayant jusqu'à 8 atomes de carbone ou un radical phényle, ou bien

$R^2$ et $R^3$ forment, conjointement avec l'atome d'azote, un noyau hétérocyclique pentagonal à

heptagonal saturé ou non saturé ayant jusqu'à deux autres hétéroatomes de la série S, N ou O,
ou bien ce radical alkyle ou alcényle représentant $R^1$ est substitué par un radical phényle qui peut lui-même être substitué jusqu'à cinq fois, égales ou différentes, par un halogène,

et leurs sels acceptables du point de vue physiologique.

**2.** Composés de formule générale (I) suivant la revendication 1, dans laquelle

A, B et D sont égaux ou différents et représentent de l'hydrogène, du fluor, du chlore, un groupe hydroxy, trifluorométhyle, trifluorométhoxy ou un groupe alkyle ou alkoxy linéaire ou ramifié ayant chacun jusqu'à 6 atomes de carbone,

E est de l'hydrogène ou un groupe alkyle linéaire ou ramifié ayant jusqu'à 4 atomes de carbone,

$R^1$ est de l'hydrogène ou représente
un groupe cyclopropyle, cyclopentyle, cyclohexyle ou 2-tétrahydropyrannyle, ou bien
un groupe acyle linéaire ou ramifié ayant jusqu'à 6 atomes de carbone, ou bien
un groupe alkyle ou alcényle linéaire ou ramifié ayant chacun jusqu'à 8 atomes de carbone, qui sont substitués le cas échéant par du fluor, un radical hydroxy, carboxy, alkoxycarbonyle linéaire ou ramifié ayant jusqu'à 4 atomes de carbone ou par un groupe de formule $-NR^2R^3$,
dans laquelle

$R^2$ et $R^3$ sont égaux ou différents et représentent de l'hydrogène ou un groupe alkyle linéaire ou ramifié ayant jusqu'à 6 atomes de carbone, ou bien

$R^2$ et $R^3$ forment, conjointement avec l'atome d'azote, un noyau de morpholine ou de pipérazine,
ou bien ce groupe alkyle représentant $R^1$ est substitué par un radical phényle qui peut être substitué de son côté jusqu'à cinq fois, égales ou différentes, par du fluor, du chlore ou du brome,

et leurs sels acceptables du point de vue physiologique.

**3.** Composés de formule générale (I) suivant la revendication 1, dans laquelle

A, B et D sont égaux ou différents et représentent de l'hydrogène, du fluor, du chlore ou un groupe alkyle ou alkoxy linéaire ou ramifié ayant chacun jusqu'à 4 atomes de carbone,

E est de l'hydrogène, un groupe méthyle ou éthyle,

$R^1$ est de l'hydrogène ou représente
un groupe cyclopropyle ou 2-tétrahydropyrannyle, ou bien
un groupe acyle linéaire ou ramifié ayant jusqu'à 4 atomes de carbone, ou bien
un groupe alkyle ou alcényle linéaire ou ramifié ayant chacun jusqu'à 6 atomes de carbone, qui sont substitués le cas échéant par un radical hydroxy, carboxy, fluoro, méthoxycarbonyle,
éthoxycarbonyle, propoxycarbonyle ou par un groupe de formule $-NR^2R^3$,
dans laquelle

$R^2$ et $R^3$ sont égaux ou différents et représentent de l'hydrogène ou un radical alkyle linéaire ou ramifié ayant jusqu'à 4 atomes de carbone,
ou bien

$R^2$ et $R^3$ forment, conjointement avec l'atome d'azote, un noyau de morpholine,
ou bien ce groupe alkyle représentant $R^1$ est substitué par un radical phényle qui peut être substitué de son côté jusqu'à cinq fois, égales ou différentes, par du fluor ou du chlore,

et leurs sels acceptables du point de vue physiologique.

**4.** Procédé de production des composés de l'invention de formule générale (I) suivant la revendication 1, caractérisé en ce qu'on fait réagir des composés de formule générale (III)

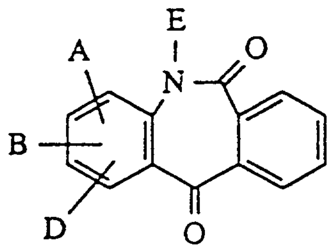

(III)

dans laquelle

A, B, D et E ont la définition indiquée ci-dessus,

tout d'abord avec des hydroxylamines de formule générale (IV)

$H_2N$-O-$R^1$ (IV)

dans laquelle

$R^1$ a la définition indiquée ci-dessus,

dans des solvants inertes, éventuellement en présence d'une base puis on transforme avec le réactif de Lawesson la fonction carbonyle voisine du groupe -N-E en une fonction thiocarbonyle et on fait varier le cas échéant les substituants A, B, D et $R^1$ par des procédés chimiques classiques et, au cas où E ne représente pas de l'hydrogène, on effectue une alkylation également par des procédés connus.

5. Médicament contenant un ou plusieurs des composés suivant les revendications 1 à 4.

6. Utilisation des composés suivant les revendications 1 à 4 pour la préparation de médicaments.